# EUROPÄISCHE PATENTSCHRIFT

(11) **EP 1 140 238 B1**
(45) Veröffentlichungstag und Bekanntmachung des Hinweises auf die Patenterteilung: **06.11.2002**
(21) Anmeldenummer: 99969222.1
(22) Anmeldetag: 21.12.1999
(51) Int. Cl.: A61L 27/34

(54) **FLUORPOLYMER-BESCHICHTUNG UND VERFAHREN ZU IHRER HERSTELLUNG**
FLUOROPOLYMER COATING AND METHOD FOR THE PRODUCTION THEREOF
REVETEMENT CONSTITUE DE POLYMERE FLUORE ET SON PROCEDE DE PRODUCTION

(30) Priorität: 21.12.1998 AT 211998
(43) Veröffentlichungstag der Anmeldung: 10.10.2001
(73) Patentinhaber: Heitz, Johannes, 4040 Linz (AT)
(72) Erfinder: HEITZ, Johannes, A-4040 Linz (AT); BÄUERLE, Dieter, A-4203 Altenberg (AT); ARENHOLZ, Enno, A-4040 Linz (AT)
(86) Internationale Anmeldenummer: EP9910229
(87) Internationale Veröffentlichungsnummer: WO00037122

(56) Entgegenhaltungen:
- WO-A-98/53767
- WO-A-99/59172
- US-A- 5 242 706
- US-A- 5 380 298
- LI S T ET AL: "Pulsed-laser deposition of crystalline Teflon (PTFE) films" APPLIED SURFACE SCIENCE, Bd. 125, Nr. 1, 2. Januar 1998 (1998-01-02), Seiten 17-22, XP000905660
- BLANCHET G B: "DEPOSITION OF AMORPHOUS FLUOROPOLYMERS THIN FILMS BY LASER ABLATION" APPLIED PHYSICS LETTERS,US,AMERICAN INSTITUTE OF PHYSICS. NEW YORK, Bd. 62, Nr. 5, 1. Februar 1993 (1993-02-01), Seiten 479-481, XP000335969 ISSN: 0003-6951
- SCHWOEDIAUER R ET AL: "CHARGE STABILITY OF PULSED-LASER DEPOSITED POLYTETRAFLUOROETHYLENE FILM ELECTRETS" APPLIED PHYSICS LETTERS,US,AMERICAN INSTITUTE OF PHYSICS. NEW YORK, Bd. 73, Nr. 20, 16. November 1998 (1998-11-16), Seiten 2941-2943, XP000788599 ISSN: 0003-6951

## Beschreibung

Die Erfindung betrifft eine dünne Beschichtung auf einem Implantat, einer Prothese oder einem Instrument für den Einsatz in der Medizin, die zum Zweck der Abweisung von Ablagerungen aufgebracht wird. Die erfindungsgemäße Beschichtung zeichnet sich durch gute Haftung und gute Abriebfestigkeit aus. Sie hat eine Schichtdicke von weniger als 200 µm und besteht aus fluorhaltigen Polymeren, in die gegebenenfalls weitere Stoffe eingebettet sein können. Diese Erfindung betrifft auch ein Verfahren zur Herstellung solcher Beschichtungen.

Die Verwendung von fluorhaltigen Polymeren als dünne abweisende und chemisch inerte Beschichtungen mit geringer Reibung auf Implantaten, Prothesen oder medizinischen Instrumenten ist bekannt. So beschreibt die Patentanmeldung WO 9210532 ein Verfahren zur Herstellung von künstlichen Blutgefäßen aus Polyester, wie sie bei Bypassoperationen verwendet werden, die innen mit Fluorpolymeren beschichtet sind, um die Bildung von Thrombosen zu verringern.

Ähnliche Überlegungen gelten für andere Implantate in der Herzchirugie wie Stents oder Scharniere von Herzklappen, die ebenfalls ständig dem Blutstrom ausgesetzt sind. Die PCT-Anmeldung WO 9317077 A1 beschreibt eine mit einer Fluorpolymerschicht versehene Operationsnadel für die Chirugie, die sich wegen ihrer geringen Reibung und ihrer geringen Oberflächenenergie besonders leicht in Gewebe einstechen läßt. Neben beschichteten Instrumenten erwähnt diese Anmeldung auch beschichtete Implantate und Prothesen.

Die PCT-Anmeldung WO 8808287 A1 beschreibt abweisende intraokulare Linsen aus Plexiglas, die auch mit einer Beschichtung aus Fluorpolymeren versehen sind. Durch die Beschichtung wird eine verminderte Beschädigung der Zellen im benachbarten Gewebe durch das Implantat erreicht.

Neben beschichteten Implantaten werden auch Implantate und Prothesen eingesetzt, die vollständig aus Fluorpolymeren bestehen. So beschreibt die PCT-Anmeldung WO 9640302 A1 eine weiche Gewebeprothese, die zusätzlich mit einer flüssigkeitsaufsaugenden Schicht versehen ist, und die PCT-Anmeldung WO 9810806 A1 beschreibt ein künstliches Blutgefäß. In beiden Fällen wurden überstreckte poröse Membranen aus Polytetrafluoräthylen (PTFE) benutzt, die unter dem Handelsnamen GORE-TEX® Surgical Membran auch speziell für den Einsatz in der Chirugie vertrieben werden. Die Implantate oder Prothesen, die vollständig aus Fluorpolymeren bestehen, können aber nur dort eingesetzt werden, wo keine zu großen Ansprüche an die mechanische Stabilität bestehen.

Die bekannten und erwünschten abweisenden Eigenschaften von Beschichtungen aus langkettigen Fluorpolymeren erschweren gleichzeitig ihre gute Haftung zu den jeweiligen Substraten, was in der Praxis bedeutet, daß sich die Schichten im Laufe der Zeit abnützen. Alternativ dazu verwendet man Beschichtungen aus sehr kurzkettigen oder stark modifizierten Fluorpolymeren, die aber häufig nur eine schlechten Kohäsion aufweisen, was ebenfalls die Abnützung begünstigt. Besonders die Verwendung von langkettigem Polytetrafluoräthylen (PTFE, Handelsname zum Beispiel TEFLON®) oder Fluoräthylenpropylen (FEP, Handelsname zum Beispiel TEFLON FEP®), die sich durch besonders gute Eigenschaften auszeichnen, war bisher praktisch nicht möglich, da sich diese Materialien nicht in Lösungsmitteln lösen, und deshalb die gängigen naßchemischen Beschichtungsverfahren ausgeschlossen sind.

In der PCT-Anmeldung WO 9317077 A1 werden deshalb lösliche Fluorpolymere (Handelsnamen TEFLON AF® oder FLUORINERT®) verwendet. Die diese Löslichkeit bedingenden chemischen Änderungen dieser Materialien führt aber zu einer deutlich geringeren chemischen Stabilität im Vergleich zu PTFE und FEP.

In den Patenschriften US 4 743 327 A, der PCT-Anmeldung WO 8808287 A1 und der deutschen Offenlegungsschrift DE 31 25 072 A1 wird als Methode zur Herstellung der Beschichtungen die Plasmapolymerisation aus gasförmigen Ausgangsmaterialen beschrieben. Die erzeugten Beschichtungen bestehen nicht aus langkettigen linearen Polymeren sondern aus relativ kurzen Ketten mit einem hohen Verzweigungs- und Vernetzungsgrad und enthalten noch viele nicht abgesättigte Bindungen. Deshalb sind sie chemisch nicht so stabil wie PTFE oder FEP.

Die in der PCT-Anmeldung WO 9317077 A1 beschriebenen Beschichtungen bestehen aus kleinen PTFE-Partikeln, die mit einem Klebemittel auf das Substrat aufgeklebt werden. Bekanntermaßen läßt sich PTFE allerdings nur schlecht verkleben.

Oft möchte man auf die Oberfläche der Fluorpolymerschicht weitere pharmakologisch wirksame Gruppen aufbringen. Dabei werden Fluor-Atome durch hydrophile Gruppen ersetzt. Dies kann entweder durch UV-Bestrahlung in einem reaktiven Medium (siehe z.B. J. Heitz et al.: *Chemical surface modification on polytetrafluorethylene films by vacuum ultraviolet excimer lamp irradiation in ammonia gas atmosphere,* Appl. Phys. Lett. **68**, 2648 (1996)) oder durch eine Plasmabehandlung mit einem geeigneten Reaktionsgas geschehen (siehe z.B. die PCT-Anmeldung WO 9810806 A1). An diese hydrophilen Gruppen werden dann die Wirkstoffe gebunden (siehe ebenfalls PCT-Anmeldung WO 9810806 A1 und Ki Dong Park et al.: *Synthesis and Characterization of SPUU-PEO-Heparin Graft Copolymers, J.* Polym. Sci. **29**, 1725 (1991)).

Neben Fluorpolymer-Schichten, die aus gasförmigen Substanzen (z.B. bei der Methode der Plasmapolymerisation) oder aus flüssigen Lösungen oder Suspensionen (z.B. das kommerziell erhältliche Sprühteflon) hergestellt werden, gibt es eine Reihe von Ansätzen zur Herstellung von Fluorpolymer-Schichten durch trockene Verfahren, wie ausgehend von einem festen homogenen PTFE-Target das Elektronenstrahlverdampfen (W. de Wilde, Thin Solid Films, Vol. 24, 101 (1974)), das Ionensputtern (I. H. Pratt, T. C. Lausman, Thin Solid Films, Vol. 10, 151 (1972)) oder die gepulste Laserdeposition (G. B. Blanchet, Appl. Phys. Lett., Vol. 62, 479 (1993)). Die so hergestellten Filme sind aber sehr rauh und sehr porös. Die Filme unterscheiden sich bezüglich ihrer Infrarotspektren und ihrer dielektrischen und elektrischen Eigenschaften von kommerziellem langkettigen PTFE-Materialien. Verglichen mit diesen weisen sie ähnlich wie die durch Plasmapolymerisation hergestellten Filme einen hohen Vernetzungs- und Verzweigungsgrad auf.

Aus der Druckschrift S. T. Li, E. Arenholz, J. Heitz, D. Bäuerle, *Pulsed-laser deposition of crystalline Teflon (PTFE) films,* Appl. Surf. Sci., Vol. 125, 17-22 (1998) ist zu entnehmen, daß PTFE-Schichten auch mit der Methode der gepulsten Laserdeposition hergestellt werden können. Als Ausgangsmaterial wird dabei ein Target aus gepreßtem PTFE-Pulver eingesetzt, das durch Laserlicht abgetragen und auf einem Substrat abgeschieden wird. Die so erzeugten Schichten sind glatt, zusammenhängend und transparent. Sie weisen ähnliche Infrarotspektren wie kommerzielles PTFE auf und haben ähnliche dielektrische und elektrische Eigenschaften. Sie weisen jedoch nicht die für medizinische Zwecke erforderliche Abriebfestigkeit auf.

Der Erfindung liegt die Aufgabe zugrunde, eine Fluorpolymer-Beschichtung der eingangs genannten Art mit verbesserter Haftung und verbesserter Abriebfestigkeit zu schaffen, die insbesondere auf medizinischem Gebiet einsetzbar ist. Außerdem liegt der Erfindung die Aufgabe zugrunde, ein Verfahren zur Herstellung einer derartigen Beschichtung zu schaffen.

Diese Aufgabe wird erfindungsgemäß mit den Merkmalen des Patentanspruchs 1 sowie mit den Verfahrensschritten des Patentanspruchs 4 gelöst.

Die Beschichtung gemäß Anspruch 1 weist eine Schichtdicke von weniger als 200 µm auf, so daß gewährleistet ist, daß die damit beschichteten Gegenstände bzw. Instrumente eine von der Beschichtung im wesentlichen unabhängige Formstabilität aufweisen. Dies ist insbesondere bei Prothesen, beispielsweise zum Ersatz von Gelenken wichtig, da diese Teile exakt zusammenpassen müssen. Durch einen Aufbau der Prothese aus massivem Fluorpolymer könnte die erforderliche Maßgenauigkeit der Prothesen nicht garantiert werden, so daß man auf formstabile Grundmaterialien wie beispielsweise Metalle angewiesen ist. Die Beschichtung dieser Grundkörper mit Fluorpolymeren ist wichtig, um die erforderliche chemische Inertheit sicherzustellen. Dabei ist es wichtig, daß die Beschichtung gut auf dem Grundkörper haftet und eine gute Abriebfestigkeit aufweist, da anderenfalls beim Einsatz der Prothese im Körper die Gefahr bestünde, daß das Grundmaterial der Prothese zum Vorschein kommt, was wiederum zu unerwünschten chemischen Reaktionen im Körper führt. Außerdem kann abgeriebenes Beschichtungsmaterial zu negativen Auswirkungen im Körper führen. Um die erwünschte gute Haftung der Beschichtung auf dem Grundkörper bei gleichzeitig guter Abriebfestigkeit zu erzielen, wird gemäß Anspruch 1 vorgeschlagen, diese durch die Methode der gepulsten Laserdeposition aus fluorhaltigen Polymeren herzustellen. Dies stellt sicher, daß keinerlei Fremdstoffe in die Beschichtung eingebaut werden, die die chemische Stabilität der Beschichtung beeinträchtigen könnten. Aufgrund der chemischen Vorgänge im Körper führt eine chemische Instabilität der Beschichtung zu Degradationserscheinungen, die wiederum die Abriebfestigkeit der Beschichtung beeinträchtigen. Viele der mit der Methode der gepulsten Laserdeposition hergestellten Fluorpolymer-Schichten weisen jedoch eine unzureichende Haftung bzw. Abriebfestigkeit auf, so daß sie für den Einsatz in der Medizin ungeeignet sind. Es hat sich jedoch überraschenderweise herausgestellt, daß Fluorpolymer-Schichten mit einer Kettenlänge von nicht weniger als der Hälfte, vorzugsweise Dreiviertel der Kettenlänge im Ausgangsmaterial und einem Vernetzungsgrad von nicht mehr als dem Doppelten, vorzugsweise dem Eineinhalbfachen des Vernetzungsgrads im Ausgangsmaterial eine sehr gute Haftung bei gleichzeitig guter Abriebfestigkeit aufweisen.

Enthält das fluorhaltige Polymer gemäß Anspruch 2 Tetrafluorethylengruppen, so ergibt sich eine besonders hohe chemische Stabilität der Beschichtung, was insbesondere für den Einsatz in der Medizin wichtig ist. Vorzugsweise besteht das fluorhaltige Polymer vollständig aus Tetrafluorethylengruppen, wodurch eine höchstmögliche Stabilität der Beschichtung erzielt wird.

Sind in der Beschichtung gemäß Anspruch 3 zusätzlich pharmakologische Wirkstoffe eingebettet, so kann die medizinische Verträglichkeit der Beschichtung weiter verbessert werden. Bei diesen Wirkstoffen kann es sich um Mittel zur weiteren Verminderung der Thrombosenbildung (z.B. Heparin), um antibiotische oder antibakterielle Wirkstoffe oder um deren Salze oder Mischungen handeln. Um diese Stoffe auf der Oberfläche zu verankern, ersetzt man zunächst an der Oberfläche des Fluorpolymers Fluor-Atome durch hydrophile Gruppen. Diese dienen dann als Ausgangspunkte zur Befestigung der Wirkstoffe, bzw. für die Befestigung von Spacern an denen dann wiederum die Wirkstoffe angebracht werden. Insbesondere ist auch daran gedacht, Wirkstoffe einzubetten, die die Immunabwehr hemmen, so daß die Beschichtung verträglicher wird.

In dem Verfahren gemäß Anspruch 4 wird ein fluorhaltiges Polymerpulver zu einem Target verpreßt. Dieses Target wird anschließend einer Temperaturbehandlung im Bereich des Schmelzpunktes des Polymers ausgesetzt, wodurch das Pulver gesintert wird. Dabei verschmelzen benachbarte Pulverkörner an ihren Rändern, was für die Beschichtungserstellung von besonderer Bedeutung ist. Insbesondere hat sich herausgestellt, daß Beschichtungen, bei denen Bulkmaterial als Target eingesetzt wird, eine sehr schlechte Adhäsion aufweisen. Targets aus verpreßtem, jedoch ungesintertem Polymerpulver führen zum Einabu ganzer Körner in die Beschichtung, so daß diese eine schlechte Kohäsion aufweist. Außerdem tritt in diesem Fall eine erhöhte Verschmutzung der Kammer ein. Überraschenderweise ergeben Beschichtungen hergestellt aus Targets aus verpreßtem und anschließend gesintertem Polymerpulver optimale Haftungen und Abriebfestigkeiten, obwohl zu vermuten ist, daß die Eigenschaften dieses gesinterten Targets zwischen denen von Bulkmaterial und von verpreßtem Pulver liegen müßten. Das gesinterte Target wird einer gepulsten Laserstrahlung ausgesetzt, deren Wellenlänge so gewählt ist, daß sie vom Polymer absorbiert werden kann. Unter dem Einfluß dieser Laserstrahlung wird das Targetpolymer allmählich abgetragen und auf einem Substrat abgeschieden, was üblicherweise als Laserdeposition bezeichnet wird. Vorzugsweise befindet sich das Substrat annähernd gegenüber dem Target, so daß die abgetragenen Polymerpartikel optimal nutzbar sind. Es hat sich herausgestellt, daß mit diesem Verfahren hergestellte Beschichtungen langkettige Polymere mit geringem Vernetzungsgrad ergeben, so daß diese Beschichtungen ein gutes Haftvermögen bei gleichzeitig guter Abriebfestigkeit aufweisen.

Gemäß Anspruch 5 ist es vorteilhaft, wenn die Beschichtungen auf Substraten abgeschieden werden, deren Temperatur höher als der Schmelzpunkt des Polymers ist. Der Schmelzpunkt von PTFE liegt bei etwa 300° C. Die damit hergestellten Beschichtungen besitzen eine sehr gute Abriebfestigkeit bei gleichzeitig sehr guter Haftung. Außerdem benötigt dieses Verfahren nur eine relativ kurze Zeit, da insbesondere keinerlei Nachbehandlungsschritte nach dem Erstellen der Beschichtung erforderlich sind.

**Alternativ** können die Beschichtungen gemäß Anspruch 6 auch bei Substrattemperaturen unterhalb der Schmelztemperatur des Polymers abgeschieden und die beschichteten Substrate in einem weiteren Verfahrensschritt auf eine Temperatur oberhalb des Schmelzpunktes von PTFE erhitzt werden. Auf diese Weise hergestellte Beschichtungen sind gegenüber Temperaturen von bis zu etwa 400°C beständig, so daß diese Temperaturbehandlung problemlos durchgeführt werden kann. Dem gegenüber zeigen Beschichtungen, die aus normalen durchgeschmolzenen Targets durch Laserdeposition hergestellt wurden, daß sie bei den hier beschriebenen Temperaturen, insbesondere aber oberhalb von 340° C, größtenteils verdampfen.

Um die Haftung der so hergestellten Filme auf Substraten zu untersuchen, wurden Beschichtungen durch verschiedene Methoden auf kristallinem Silizium abgeschieden. Die Haftung 'der Beschichtungen auf diesem Substrat wurde durch einen "Scotch-Tape-Test" bestimmt. Bei diesem Test wird die Haftung qualitativ durch Abziehen eines aufgeklebten Klebebandes bestimmt. Hierzu wurden gängige Klebebänder der Marke "Scotch® Magic® Tape", die von der Firma 3M hergestellt werden, verwendet.

Das Ergebnis dieser Untersuchungen ist in der Tabelle 1 übersichtlich zusammengefaßt und wird im folgenden erläutert. Bei kommerziellen freitragenden PTFE-Folien (A) - Foliendicke 25 µm, Bezugsquelle Fa. Goodfellow - lassen sich im "Scotch-Tape-Test" aufgeklebte Klebebänder glatt abziehen, ohne daß Material daran haften bleibt. Diese PTFE-Folie haftet naturgemäß jedoch nicht auf einem darunterliegenden Substrat, da praktisch keine Adhäsion vorhanden ist.

Dagegen zeigen Beschichtungen (B), die aus auf einem Substrat aufgesprühten Schichten aus Teflonspray (z.B. Tygaflor®) durch Verdampfen des Lösungsmittels hergestellt wurden, im Scotch-Tape-Test Kohäsionsversagen in der Fluorpolymerschicht. Bei Sprühteflon handelt es sich um sehr kurze PTFE-Ketten, die z.B. in Trichlorethan löslich sind. Kohäsionsversagen bedeutet hier, daß die Kohäsion der Fluorpolymerschicht so gering war, daß ein Teil der Schicht mit dem Klebeband abgezogen wurde, während gleichzeitig der größere Teil der Schicht auf dem Substrat verblieb. Nach dem Abziehen war die gesamte Klebefläche, die mit der Beschichtung in Kontakt gekommen war, mit einem dünnen Fluorpolymer-Schicht bedeckt. Bei nochmaliger Durchführung des Versuchs auf demselben Bereich der Probe trat das gleiche Ergebnis auf.

Beschichtungen, die durch Plasmapolymerisation aus einem Prozeßgas (C) oder durch gepulste Laserdeposition aus homogenen PTFE-Targets (D) hergestellt wurden zeigten im "Scotch-Tape-test" ebenfalls Kohäsionsversagen. Schichten mit geringer Kohäsion weisen schlechte Abriebfestigkeit bei mechanischer Beanspruchung auf. Über die Adhäsion an der Grenzfläche zwischen der Fluorpolymerschicht und dem Substrat kann durch diesen Test wegen der schlechten Kohäsion keine Aussage gemacht werden.

Überraschenderweise zeigen aber Beschichtungen, die nach dem erfindungsgemäßen Verfahren durch gepulste Laserdeposition aus einem Target aus gepreßtem und gesintertem PTFE-Pulver auf Siliziumsubstrate abgeschieden wurden, im Scotch-Tape-Test eine gute bis sehr gute Kohäsion, die bei den beiden vorteilhaften Ausprägungen des Verfahrens vergleichbar der von kommerziellen freitragenden Folien ist. In den beiden vorteilhaften Ausprägungen des Verfahrens ist die Adhäsion sehr gut für Substrattemperaturen beziehungsweise Nachbehandlungstemperaturen zwischen 340 - 600°C (F, H) oder abhängig von der Beschichtungsdicke gut bis sehr gut für Substrattemperaturen beziehungsweise Nachbehandlungstemperaturen zwischen dem Schmelzpunkt von PTFE und 340°C (E, G). Unter sehr guter Adhäsion ist hier zu verstehen, daß das Klebeband glatt abgezogen werden konnte, ohne daß Material auf der Klebefläche zurückblieb. Mit guter Adhäsion ist zu verstehen, daß sich Teile der Schicht mit dem Klebeband abziehen lassen, es also teilweise zum Adhäsionsversagen im Scotch-Tape-Test kommt.

Beschichtungen (I), die nach dem erfindungsgemäßen Verfahren bei Temperaturen unterhalb des Schmelzpunktes von PTFE hergestellt wurden, zeigen ohne Nachbehandlung nur noch befriedigende Adhäsion. Darunter ist zu verstehen, daß die Filme zwar auf dem Substrat haften, sich aber im Scotch-Tape-Test abziehen lassen. Allerdings ist die Kohäsion dieser Filme noch immer so gut, daß sie sich nahezu vollständig abziehen lassen. Die gute Kohäsion der erfindungsgemäßen Schichten führt zu einer guten bis sehr guten Abriebfestigkeit der Schichten, solange keine spitzen Gegenstände zum Abreiben eingesetzt werden.

Weitere Vorteile und Merkmale der vorliegenden Erfindung werden in der folgenden detaillierten Beschreibung anhand der dazugehörigen Figuren dargestellt, in denen ein Ausführungsbeispiel der vorliegenden Erfindung enthalten ist. Es sollte jedoch verstanden werden, daß die Zeichnung nur zum Zweck der Darstellung der Erfindung dient und nicht den Schutzbereich der Erfindung beschränkt.

In der Zeichnung bezeichnen gleiche Bezugszeichen stets gleiche Teile.

Es zeigt:
- Figur 1: einen kleinen Ausschnitt einer medizinischen Prothese mit einer darauf aufgebrachten erfindungsgemäßen Beschichtung und
- Figur 2: eine teilweise geschnittene Darstellung einer Vorrichtung zur Herstellung der Beschichtung

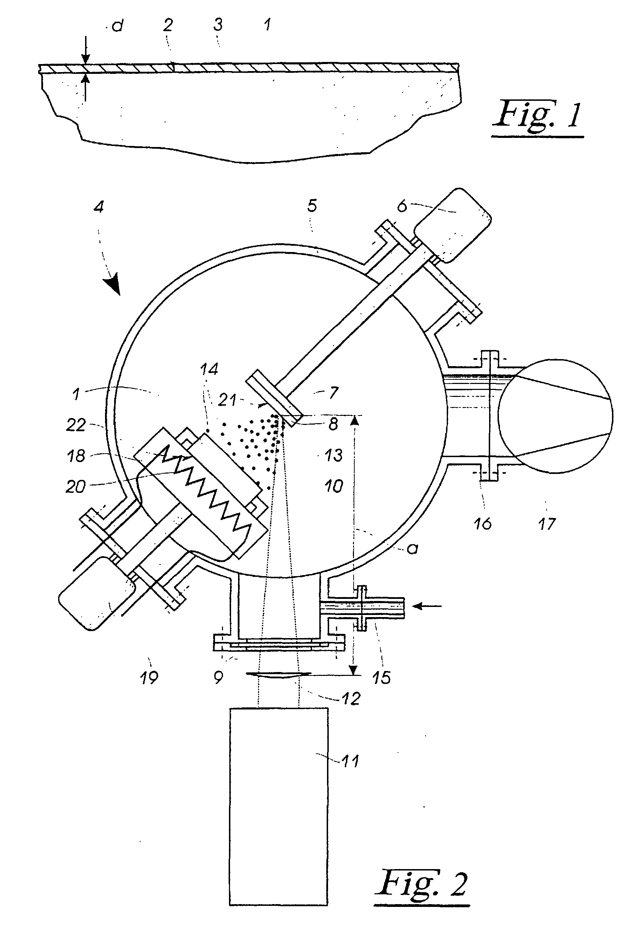

In Figur 1 ist ein kleiner Ausschnitt aus einer Prothese (1) dargestellt, die beispielsweise aus hochlegiertem Edelstahl besteht, wie er regelmäßig in der Medizin eingesetzt wird. Um die Oberfläche (2) der Prothese (1) vor chemischen Reaktionen des nicht dargestellten umgebenden Körperteils zu schützen, ist eine Beschichtung (3) aus Polytetrafluorethylen (PTFE) an der Oberfläche (2) vorgesehen. Diese Beschichtung (3) weist eine Dicke (d) zwischen 50 µm und 200 µm auf, so daß eine dichte Versiegelung der Oberfläche (2) der Prothese (1) durch die Beschichtung (3) gegeben ist. Es ist insbesondere daran gedacht, die Beschichtung (3) allseits auf der Oberfläche (2) der Prothese (1) anzubringen, damit ein umfassender Schutz der Oberfläche (2) der Prothese (1) gewährleistet ist.

Zur Herstellung der Beschichtung (3) wird eine Vorrichtung (4) gemäß Figur 2 eingesetzt. Diese Vorrichtung (4) weist im wesentlichen eine Kammer (5) auf, in der ein von einem Drehantrieb (6) in Drehung versetzter Drehteller (7) gehalten ist. Dieser Drehteller (7) ist einseitig mit einem Target (8) aus gepreßtem und gesintertem PTFE-Pulver belegt, das das Ausgangsmaterial für den Beschichtungsvorgang bildet.

In der Kammer (5) ist außerdem ein Fenster (9) vorgesehen, durch das Licht (10) eines gepulsten Lasers (11) in die Kammer (5) gelangt. Der gepulste Laser (11) kann beispielsweise ein Excimer-Laser, insbesondere ein KrF-Laser sein, wobei auch jeder andere Laser geeignet ist, dessen Licht vom Target (8) ausreichend absorbiert wird. Zur Erzielung einer ausreichend hohen Energiedichte wird das Licht (10) des gepulsten Lasers (11) mittels einer außerhalb der Kammer (5) vorgesehenen Sammellinse (12) fokusiert. Diese Lage der Sammellinse (12) ist vorteilhaft, weil hierdurch vermieden wird, daß die Sammellinse (12) versehentlich beschichtet wird. Der Abstand (a) zwischen der Sammellinse (12) und dem Target (8) ist dabei so eingestellt, daß ein Brennpunkt (13) des Lichtes (10) auf dem Target (8) zu liegen kommt. Damit wird eine maximale Energiedichte des Lichtes (10) auf dem Target (8) erzielt, wodurch das Target (8) in optimaler Weise abgetragen wird. Alternativ ist es insbesondere beim Einsatz sehr energiereicher gepulster Laser (11) auch vorstellbar, den Brennpunkt (13) geringfügig vor oder hinter dem Target (8) zu legen, um keine zu großen Energiedichten auf dem Target (8) zu erzielen. Da das Target (8) vom Drehantrieb (6) in Drehung versetzt wird, wandert der Brennpunkt (13) während der Drehung über das Target (8), wodurch Überhitzungen des Targets (8) zuverlässig vermieden werden. Wichtig dabei ist, daß der Brennpunkt (13) nicht im Bereich der Drehachse des Targets (8) auf dieses fällt, da dann der Brennpunkt (13) stets auf der gleichen Stelle das Target (8) trifft.

Zum Schutz des Fensters (9) vor abgetragenem Targetmaterial (14) wird die Kammer (5) von einem Gas durchströmt, welches an einem Einlaß (15) nahe dem Fenster (9) in die Kammer (5) einströmt und an einem Auslaß (16) von einer Vakuumpumpe (17) abgesaugt wird. Als Gas hat sich insbesondere ein Inertgas wie beispielsweise Argon bewährt, da dieses mit dem abgetragenen Targetmaterial (14) in keiner Weise chemisch reagiert. Grundsätzlich könnte aber auch jedes andere Gas eingesetzt werden.

Dem Target (8) gegenüberliegend ist ein weiterer Drehteller (18) vorgesehen, der von einem weiteren Drehantrieb (19) in Drehung versetzt wird. Der Drehteller (18) ist mit einer Heizung (20) ausgestattet, mit der eine auf dem Drehteller (18) gehaltene Prothese (1) auf die erforderliche Prozeßtemperatur aufgeheizt wird. Da der Drehteller (18) dem Target (8) gegenüberliegt, wird das abgetragene Targetmaterial (14) optimal zur Beschichtung der Prothese (1) genutzt.

Im folgenden wird anhand der Figur 2 das erfindungsgemäße Beschichtungsverfahren erläutert. Zunächst wird PTFE-Pulver zu einer Tablette verpreßt und anschließend einer Temperaturbehandlung im Bereich des Schmelzpunktes des PTFE ausgesetzt, so daß die aneinanderliegenden Kornflächen des Pulvers miteinander verschmelzen. Durch dieses als "Sintern" bezeichnete Verfahren ergibt sich ein Target (8), das durch seine Körnung eine deutlich andere Feinstruktur aufweist als massives PTFE.

Das Target (8) wird in den Drehteller (7) eingebaut, während die zu beschichtende Prothese (1) auf dem beheizbaren Drehteller (18) gehalten wird. Nun wird die Kammer (5) geschlossen, mittels der Vakuumpumpe (17) evakuiert und die zu beschichtende Prothese (1) mittels der Heizung (20) auf die gewünschte Prozeßtemperatur aufgeheizt.

Nun wird über den Einlaß (15) Inertgas in die Kammer (5) eingelassen, das permanent mit der Vakuumpumpe (17) aus der Kammer abgepumpt wird, so daß ein kontinuierlicher Gasstrom mit einem Druck zwischen 0,1 und 1,0 mbar entsteht. Dies verhindert ein versehentliches Beschichten des Fensters 9. Anschließend wird der gepulste Laser (11) eingeschaltet, so daß gepulstes Licht (10) auf das Target (8) fokusiert wird. Dadurch wird Material (14) aus dem Target (8) abgetragen, das sich vornehmlich in etwa senkrecht zur Oberfläche (21) des Targets (8) von diesem wegbewegt. Dieses abgetragene Targetmaterial (14) gelangt direkt zur zu beschichtenden Prothese (1), auf der sich dieses Material (14) niederschlägt.

Wird die Prothese (1) während des Beschichtungsvorgangs auf eine Temperatur oberhalb der Schmelztemperatur des PTFE aufgeheizt, so ergibt sich eine völlig ausreichende Beschichtungsqualität, die keinerlei Nachbehandlung erfordert. Anderenfalls kann die Prothese (1) innerhalb der Kammer (5) nach Beendigung des Beschichtungsvorganges mittels der Substratheizung (20) auf eine Temperatur oberhalb des Schmelzpunktes des PTFE aufgeheizt werden, wodurch sich eine gut haftende und abriebfeste Beschichtung (3) ergibt. Alternativ könnte dieser thermische Nachbehandlungsschritt auch außerhalb der Kammer (5) in einem gesonderten Ofen erfolgen, so daß die Kammer (5) für die Beschichtung der nächsten Prothese (1) zur Verfügung steht.

Um eine allseitige Beschichtung der Prothese (1) zu gewährleisten, ist es erforderlich, nach Beendigung des Beschichtungsvorganges die Prothese (1) zu drehen, damit auch dessen Rückseite (22) beschichtet werden kann. Dabei ist es jedoch vorstellbar, einen ggf. vorzunehmenden thermischen Nachbehandlungsschritt erst nach der Beschichtung aller Seiten der Prothese (1) vorzunehmen.

Da einige Ausführungsbeispiele der vorliegenden Erfindung nicht gezeigt bzw. beschrieben sind, ist zu verstehen, daß eine Vielzahl von Änderungen und Abwandlungen dieses beschriebenen Ausführungsbeispiels möglich ist, ohne den Schutzbereich der Erfindung zu verlassen, der durch die Ansprüche festgelegt ist.

**Tabelle 1**

| | **Beschichtung** | **Ergebnis des Scotch-Tape-Tests** | |
|---|---|---|---|
| | | **Kohäsion** | **Adhäsion** |
| **A** | Kommerzielle freitragende PTFE-Folie | sehr gut | sehr schlecht |
| **B** | Aufgesprühte Schicht aus Teflonspray | schlecht | - |
| **C** | Plasmapolymerisierte Schicht | schlecht | - |
| **D** | Aus einem homogenen PTFE-Target bei einer Substrattemperatur von 300°C durch gepulste Laserdeposition hergestellte Beschichtung | schlecht | - |
| **E** | Beschichtung hergestellt nach dem erfindungsgemäßen Verfahren gemäß Anspruch 5 bei einer Substrattemperatur von 300°C | sehr gut | gut - sehr gut |
| **F** | Beschichtung hergestellt nach dem erfindungsgemäßen Verfahren gemäß Anspruch 5 bei einer Substrattemperatur von 400°C | sehr gut | sehr gut |
| **G** | Beschichtung hergestellt nach dem erfindungsgemäßen Verfahren gemäß Anspruch 6 bei einer Substrattemperatur von 25°C und einer Nachbehandlungstemperatur von 300°C | sehr gut | gut - sehr gut - |
| **H** | Beschichtung hergestellt nach dem erfindungsgemäßen Verfahren gemäß Anspruch 6 bei einer Substrattemperatur von 25 °C und einer Nachbehandlungstemperatur von 400°C | sehr gut | sehr gut |
| **I** | Beschichtung hergestellt nach dem erfindungsgemäßen Verfahren gemäß Anspruch 4 bei einer Substrattemperatur von 25 °C ohne zusätzliche Nachbehandlung | gut | befriedigend |

### Bezugszeichenliste

- 1: Prothese
- 2: Oberfläche der Prothese
- 3: Beschichtung
- 4: Vorrichtung
- 5: Kammer
- 6: Drehantrieb
- 7: Drehteller
- 8: Target
- 9: Fenster
- 10: Licht
- 11: gepulster Laser
- 12: Sammellinse
- 13: Brennpunkt
- 14: abgetragenes Targetmaterial
- 15: Einlaß
- 16: Auslaß
- 17: Vakuumpumpe
- 18: Drehteller
- 19: Drehantrieb
- 20: Heizung
- 21: Oberfläche des Targets
- 22: Rückseite
- d: Dicke der Beschichtung
- a: Abstand der Sammellinse vom Target

## Patentansprüche

1. Abweisende Beschichtung (3) mit zumindest guter Haftung und zumindest guter Abriebfestigkeit auf einem Implantat, einer Prothese (1) oder einem Instrument für den Einsatz in der Medizin aus mindestens einem Fluorpolymer mit einer Schichtdicke (d) von weniger als 200 µm, **dadurch gekennzeichnet, daß** die Beschichtung (3) durch die Methode der gepulsten Laserdeposition aus mindestens einem verpreßten und anschließend gesinterten fluorhaltigen Polymer (8) hergestellt wird und die mittlere Kettenlänge der in der Beschichtung (3) enthaltenen Polymerketten nicht weniger als die Hälfte der Kettenlänge im Ausgangsmaterial (8) beträgt und der Vernetzungsgrad der in der Beschichtung (3) enthaltenen Polymerketten nicht mehr als das Doppelte des Vernetzungsgrades im Ausgangsmaterial (8) beträgt.

2. Beschichtung nach Anspruch 1 **dadurch gekennzeichnet, daß** das fluorhaltige Polymer (8) Tetrafluorethylengruppen enthält.

3. Beschichtung nach Anspruch 1 oder 2, **dadurch gekennzeichnet, daß** in die Beschichtung (3) mindestens ein pharmakologischer Wirkstoff eingebettet ist.

4. Verfahren zur Herstellung einer dünnen Beschichtung (3) auf einem Implantat, einer Prothese (1) oder einem Instrument für den Einsatz in der Medizin, bei dem fluorhaltiges Polymerpulver zu einem Target (8) verpreßt und anschließend mittels einer Temperaturbehandlung im Bereich des Schmelzpunktes des Polymers gesintert wird, und das Target (8) durch gepulste Laserstrahlung (10) mit Lichtwellenlängen zwischen 100 nm und 20 µm und Pulsdauern zwischen 10 fs und 1 ms wenigstens teilweise abgetragen wird, wobei das abgetragene Polymer (14) auf dem zu beschichtenden Körper (1) unter Bildung der Beschichtung (3) abgeschieden wird.

5. Verfahren nach Anspruch 4, bei dem die dünne Beschichtung (3) auf dem zu beschichtenden Körper (1) abgeschieden wird, dessen Temperatur bei der Abscheidung oberhalb der Schmelztemperatur des abgeschiedenen, fluorhaltigen Polymers und unterhalb von dessen Verdampfungstemperatur liegt.

6. Verfahren nach Anspruch 4, bei dem die dünne Beschichtung (3) auf dem zu beschichtenden Körper (1) abgeschieden wird, dessen Temperatur bei der Abscheidung unterhalb der Schmelztemperatur des abgeschiedenen fluorhaltigen Polymers liegt und nach der Abscheidung einer Temperaturbehandlung oberhalb der Schmelztemperatur und unterhalb der Verdampfungstemperatur des abgeschiedenen, fluorhaltigen Polymers unterzogen wird.

## Claims

1. Repellent coating (3) with at least good adhesion and at least good abrasion resistance on an implant, a prosthesis (1) or an instrument for use in medicine composed of at least one fluoropolymer with a layer thickness (d) of less than 200 µm, **characterized in that** the coating (3) is produced by the method of pulsed laser deposition from at least one compressed and subsequently sintered fluorine-containing polymer (8), and the average chain length of the polymer chains present in the coating (3) is not less than half the chain length in the starting material (8), and the degree of crosslinking of the polymer chains present in the coating (3) is not more than twice the degree of crosslinking in the starting material (8).

2. Coating according to Claim 1, **characterized in that** the fluorine-containing polymer (8) comprises tetrafluoroethylene groups.

3. Coating according to Claim 1 or 2, **characterized in that** at least one pharmacological active substance is embedded in the coating (3).

4. Process for the production of a thin coating (3) on an implant, a prosthesis (1) or an instrument for use in medicine, in which fluorine-containing polymer powder is compressed to a target (8) and then sintered by means of a thermal treatment in the region of the melting point of the polymer, and the target (8) is at least partly ablated by pulsed laser radiation (10) with light wavelengths between 100 nm and 20 µm and pulse durations between 10 fs and 1 ms, the ablated polymer (14) being deposited on the article (1) to be coated, with formation of the coating (3).

5. Process according to Claim 4, in which the thin coating (3) is deposited on the article (1) to be coated, the temperature of which during the deposition is above the melting point of the deposited, fluorine-containing polymer and below the vaporization temperature thereof.

6. Process according to Claim 4, in which the thin coating (3) is deposited on the article (1) to be coated, the temperature of which during the deposition is below the melting point of the deposited, fluorine-containing polymer and, after the deposition, is subjected to a thermal treatment above the melting point and below the vaporization temperature of the deposited, fluorine-containing polymer.

## Revendications

1. Revêtement hydrophobe (3) ayant au moins une bonne adhérence et au moins une bonne résistance à l'abrasion sur un implant, sur une prothèse (1) ou sur un instrument pour utilisation médicale, fait d'au moins un polymère fluoré ayant une épaisseur de couche (d) de moins de 200 µm, **caractérisé par le fait que** le revêtement (3) est fabriqué par le procédé de déposition par laser pulsé à partir d'au moins un polymère contenant du fluor, pressé et ensuite fritté (8), et **par le fait que** la longueur de chaîne moyenne des chaînes polymères contenues dans le revêtement (3) ne soit pas moins de la moitié de la longueur de chaîne dans le matériau de départ (8), et par le degré de réticulation des chaînes polymères contenues dans le revêtement (3) ne soit pas plus du double du degré de réticulation dans le matériau de départ (8).

2. Revêtement selon la revendication 1, **caractérisé par le fait que** le polymère contenant du fluor (8) comprenne des groupements tétrafluoroéthylène.

3. Revêtement selon la revendication 1 ou 2, **caractérisé par le fait que** l'on incorpore au moins un agent actif pharmacologique dans le revêtement (3).

4. Procédé de fabrication d'un revêtement mince (3) sur un implant, sur une prothèse (1) ou sur un instrument pour utilisation en médecine, au cours duquel une poudre polymère contenant du fluor est pressée pour former une cible (8) puis frittée à l'aide d'un traitement thermique dans le domaine du point de fusion du polymère, et au cours duquel la cible (8) est au moins partiellement éliminée par un rayonnement laser pulsé (10) ayant des longueurs d'onde lumineuse comprises entre 100 nm et 20 µm et des durées d'impulsion entre 10 fs et 1 ms, le polymère éliminé (14) étant déposé sur le corps à revêtir (1) avec formation du revêtement (3).

5. Procédé selon la revendication 4, au cours duquel le revêtement mince (3) est déposé sur le corps à revêtir (1), dont la température, lors du dépôt, se situe au-dessus de la température de fusion du polymère déposé, contenant du fluor, et au-dessous de sa température de vaporisation.

6. Procédé selon la revendication 4, au cours duquel le revêtement mince (3) est déposé sur le corps à revêtir (1), dont la température, lors du dépôt, se situe au-dessous de la température de fusion du polymère déposé, contenant du fluor, et au cours duquel, après le dépôt, le revêtement est soumis à un traitement thermique au-dessus de la température de fusion et au-dessous de la température de vaporisation du polymère déposé, contenant du fluor.
